(19) Europäisches Patentamt
European Patent Office
Office européen des brevets

(11) Veröffentlichungsnummer : **0 061 672**
**B1**

(12) # EUROPÄISCHE PATENTSCHRIFT

(45) Veröffentlichungstag der Patentschrift : **13.06.84**

(51) Int. Cl.³ : **C 07 C 69/96**, C 07 C 68/00

(21) Anmeldenummer : **82102282.9**

(22) Anmeldetag : **19.03.82**

(54) **Verfahren zur Herstellung von Kohlensäureestern.**

(30) Priorität : **31.03.81 DE 3112794**

(43) Veröffentlichungstag der Anmeldung : **06.10.82 Patentblatt 82/40**

(45) Bekanntmachung des Hinweises auf die Patenterteilung : **13.06.84 Patentblatt 84/24**

(84) Benannte Vertragsstaaten : **CH DE FR GB IT LI**

(56) Entgegenhaltungen :
**EP-A- 0 013 957**
**EP-A- 0 013 958**
**EP-A- 0 041 622**

(73) Patentinhaber : **BAYER AG**
**Konzernverwaltung RP Patentabteilung**
**D-5090 Leverkusen 1 Bayerwerk (DE)**

(72) Erfinder : **Heitz, Walter, Prof. Dr.**
**Am Schmidtborn**
**D-3575 Kirchhain (DE)**
Erfinder : **Füllmann, Heinz**
**Am Richtsberg 88/405**
**D-3550 Marburg / Lahn (DE)**
Erfinder : **Ball, Peter, Dr.**
**Kettlerstrasse 11**
**D-8263 Emmerting (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 061 672 B1

**Beschreibung**

Gegenstand der vorliegenden Erfindung ist ein Verfahren zur Herstellung von Kohlensäureestern aus Monoalkoholen und Harnstoffen im Molverhältnis von mindestens 2 : 1 (Monoalkohol : Harnstoff) oder aus Monoalkoholen und Urethanen im Molverhältnis von mindestens 1 : 1 (Monoalkohol : Urethangruppe) in Gegenwart von Katalysatoren, das dadurch gekennzeichnet ist, daß man die Umsetzung bei Temperaturen zwischen 120 °C und 270 °C, vorzugsweise zwischen 140 °C und 230 °C in Gegenwart eines Katalysators der folgenden Formel I durchführt.

$$R^1_{n_1}-\underset{(OH)_{n_4}}{\overset{X_{n_2}}{M}}-(O-R^2)_{n_3} \qquad (I)$$

In Formel I sind
M = Si, Ge, oder Sn,
$R_1$ = $C_1$-$C_{18}$-Alkyl oder $C_6$-$c_{20}$-Aryl,
$R_2$ = $C_1$-$C_{18}$-Alkyl, $C_6$-$C_{20}$-Aryl oder $C_2$-$C_{18}$-Acyl,
X = Halogen, —SH, = $O/_2$ oder = $S/_2$
$n_1$, $n_2$, $n_3$ und $n_4$ jeweils eine ganze Zahl von 0 bis 4 und $n_1 + n_2 + n_3 + n_4 = 4$,
wobei für M = Sn $n_1$ nicht 0 ist ;
in Formel I kann außerdem die Summe
$n_1 + n_2 + n_3 + n_4 = 2$ sein, wobei allerdings
$n_2$ nicht 2 sein darf ;
in Formel I kann auch M = As, sein, wobei die Summe aus $n_1 + n_2 + n_3 + n_4$ die Werte 3 oder 5 hat ;
in Formel I kann auch M = S, Se oder Te sein, wobei die Summe aus $n_1 + n_2 + n_3 + n_4$ die Werte 4 oder 6 hat.

In Formel I , ist X vorzugsweise Cl, $R_1$ vorzugsweise $C_1$-$C_8$-Alkyl oder Phenyl, $R_2$ vorzugsweise $C_1$-$C_8$-Alkyl, Phenyl oder $-\underset{\underset{O}{\|}}{C}-(C_1-C_8)$-Alkyl.

Aus den deutschen Offenlegungsschriften DE-OS 2 903 506 und DE-OS 2 903 507 ist die katalytische Umsetzung von Harnstoffen bzw. Urethanen mit Alkoholen bekannt. Als Katalysatoren der 4. bis 6. Hauptgruppe des Periodischen Systems der Elemente (siehe Handbook of Chemistry and Physics) werden in diesen Offenlegungsschriften nur Orthostannate, Amine, Phosphine, Phosphinoxide, Thioether und Thiolate aufgeführt. Nachteilig sind hierbei die teilweise langen Reaktionszeiten, sofern sie einzeln und nicht in Kombination eingesetzt werden.

In Zeitschrift für Naturforschung (Bd. 1, Seite 518 bis 523, 1946) wird die Umsetzung von Harnstoff unter Katalyse von Schwermetallsalzen schwacher organischer Säuren zu Urethanen beschrieben. Als Katalysatoren werden im einzelnen unter anderem Zinn (IV) chlorid, Bleiacetat eingesetzt. Außerdem wird im entsprechenden DE-PS 753 127 unter anderem $SnCl_4$, $SnCl_6$ $(NH_4)_2$, $SnCl_2$ bzw. Pb $(OCO—CH_3)_4$ eingesetzt.

Nach der Lehre dieser beiden Literaturstellen werden nur in Spuren Carbonat gebildet, sofern man die Reaktionszeit verlängert. (Siehe Ang. Chem., 92 (1980) Seite 742-743).

Die Menge an Katalysator für die erfindungsgemäße Umsetzung beträgt im allgemeinen zwischen $10^{-3}$ bis 5 Mol-%, bezogen auf Mole an jeweils eingesetzten Harnstoffen oder Urethanen ; vorzugsweise werden zwischen $10^{-2}$ bis 1 Mol-% eingesetzt.

Das Molverhältnis Monoalkohol : Harnstoff beträgt mindestens 2 : 1, vorzugsweise 3 : 1 bis 4 : 1 ; das Molverhältnis Monoalkohol : Urethangruppe beträgt mindestens 1 : 1, vorzugsweise 3 : 1 bis 4 : 1.

Anstelle oder zusätzlich zu den Monoalhoholen kann man die erfindungsgemäße Umsetzung auch mit Di-alkoholen, Tri-alkoholen oder anderen Polyalkoholen in entsprechender Weise durchführen. Bei geeigneter Wahl der Molverhältnisse von Polyalkohol zu Harnstoff bzw. Urethan und gegebenenfalls Monoalkohol erhält man höhermolekulare Kohlensäureester, d. h. Polycarbonate. (Siehe beispielsweise DE-OS 2 903 506 und 2 903 507). So beträgt beispielsweise im Falle der Dialkohole das Molverhältnis Dialkohol : Harnstoff 1 : 1 und Dialkohol : Urethangruppe 0,5 : 1.

Als Monoalkohole bzw. Polyalkohole kommen vorzugsweise primäre Alkohole in Betracht, die eine beliebige Anzahl von C-Atomen enthalten können. Geeignete Alkohole sind aliphatische, cycloaliphatische, araliphatische und heterocyclische Alkohole. Polyalkohole im Sinne vorliegender Erfindung sind beispielsweise solche mit 2 bis 3 alkoholischen OH-Gruppen, wobei bei entsprechendem Abstand der alkoholischen OH-Gruppen im Molekül cyclische Kohlensäureester entstehen können.

Die geeigneten Monoalkohole haben vorzugsweise 1 bis 20 C-Atome.

Beispiele für Monoalkohole sind Methanol, Ethanol, Propanole, Butanole, Pentanole, Hexanole, Oktanole, Stearylalkohol, Methylolcyclohexan, Benzylalkohol, 2-Phenylethanol, 2-Naphthylethanol, Furfurylalkohol etc.

Geeignete Polyalkohole haben vorzugsweise 5 bis 20 C-Atome.

Beispiele für Polyalkohole sind Hexan-1,6-diol, Decandiole, 1,4-Dimethylolcyclohexan, 1,4-Dimethylolbenzol, 3(4), 8(9) Bis (Hydroxymethyl)-tricyclo-/5,2,1,$0^{2:6}$/)-decan

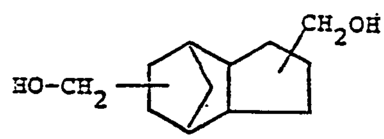

und Bis-hydroxymethyl-tetrahydrofurane etc.

Erfindungsgemäß geeignete Harnstoffe sind neben Harnstoff selbst die durch Kohlenwasserstoffreste mono-, di-, tri- oder tetra-substituierte Harnstoffe, vorzugsweise die der folgenden Formel II

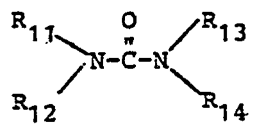

(II)

worin $R_{11}$ bis $R_{14}$ H oder Kohlenwasserstoffreste mit 1 bis 18 C-Atomen sind, die entweder Heteroatome wie O, N oder S enthalten können oder zu heterocyclischen Ringen verbunden sein können, wobei nur Verknüpfungen von $R_{11}$ mit $R_{12}$ und/oder $R_{13}$ mit $R_{14}$ in Frage kommen und wobei die bei der Umsetzung entstehenden Amine einen Siedepunkt von unter 150 °C, vorzugsweise unter 120 °C haben müssen. $R_{11}$ bis $R_{14}$ können sowohl gleiche als auch verschiedene Bedeutung haben.

Als Kohlenwasserstoffreste kommen Alkyle, Alkoxyalkyle, Aryle, Aralkyle und Cycloalkyle in Frage.

Beispiele für erfindungsgemäß geeignete Harnstoffe sind Harnstoff, Mono-, Di-, Tri- und Tetramethylharnstoffe, Mono-, Di-, Tri- und Tetraethylharnstoffe, Mono- und Di-phenylharnstoffe, Benzylharnstoffe, Cyclohexylharnstoffe etc.

Erfindungsgemäß geeignete Urethane sind beispielsweise Monourethane oder Bisurethane, insbesondere jedoch solche primärer Alkohole.

Erfindungsgemäß bevorzugt geeignete Urethane sind solche der Formeln IIIa und IIIb

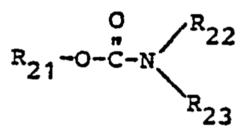

(IIIa)

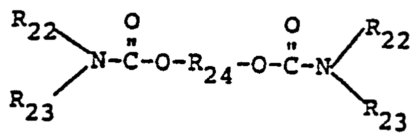

(IIIb)

worin

$R_{21}$ ein primäres, gegebenenfalls substituiertes $C_1$-$C_{18}$-Alkyl ist, das auch cycliste Strukturen sowie Heteroatome enthalten kann,

$R_{22}$ und $R_{23}$ gleich oder verschieden sein können und H oder $C_1$-$C_{18}$-Alkyl, vorzugsweise jedoch H sind, und

$R_{24}$ der von einem diprimären Dialkohol unter Abspaltung der beiden OH-Gruppen abgeleitete zweibindige Rest mit vorzugsweise 2 bis 20 C-Atomen ist, der auch cyclische Strukturen enthalten kann.

Erfindungsgemäß geeignete Urethane der Formel IIIa sind beispielsweise Iso-octylurethan (Carbamidsäureisooctylester), Hexylurethan, Butylurethan (Carbamidsäurebutylester) oder (N-Butyl)isooctylurethan (N-Butyl-carbamidsäureisooctylester).

Erfindungsgemäß geeignete Urethane der Formel IIIb sind beispielsweise 1,6-Hexandiol-bis-urethan, 1,10-Decandiol-bisurethan oder 1,6-hexandiol-bis-(N-butyl)-urethan.

Erfindungsgemäß geeignete Katalysatoren der Formel I sind beispielsweise die folgenden :

Dimethyldichlorsilan, Trimethylchlorsilan, Siliciumdioxid, Dibutyldimethoxyzinn, Dibutylzinnoxid, Arsentrioxid, Zeolithe.

Zur Durchführung der erfindungsgemäßen Reaktion werden die Komponenten, d. h. Harnstoff, bzw. Urethane, Alkohol und Katalysator zusammen unter Rühren auf Temperaturen von mindestens 120 °C erwärmt, während 1 bis 10 Stunden, vorzugsweise 2 bis 5 Stunden, auf Temperaturen bis zu 270 °C erwärmt und ausreagieren lassen.

Anschließend wird der Ansatz bei niedrigerer Temperatur (bis etwa 120 °C) in Vakuum (bis etwa 0,05 Torr) schonend destillativ getrennt und der Kohlensäureester isoliert, wobei die Ausbeuten im allgemeinen gut sind.

Die nach dem erfingungsgemäßen Verfahren hergestellten Kohlensäureester sind bekannte Verbindungen und eignen sich bekanntlich als Lösungsmittel in der Organischen Chemie sowie als Zwischen- bzw. Ausgangsprodukte für die verschiedensten chemischen Reaktionen.

Insbesondere eignen sie sich in bekannter Weise (vgl. DT-PS 1 031 512, Beispiel 1) zur Synthese von Polycarbonaten durch Umsetzung mit Diolen.

### Beispiel 1

12,0 g Harnstoff, 65,0 g 2-Ethylhexanol und 0,5 g Dibutylzinnoxid wurden zusammen erwärmt. Bei ca. 145 °C begann die Mischung zu sieden. Während einer halben Stunde wurde die Reaktionstemperatur auf 190 °C erhöht ; zu diesem Zeitpunkt waren ca. 50 % der theoretisch möglichen Ammoniakmenge abgespalten. Die Reaktionstemperatur wurde nun im Verlauf von 3,5 Stunden bis auf 230 °C erhöht ; es waren dann insgesamt 6,46 g Ammoniak abgeschieden. Anschließend wurde die Reaktionsmischung fraktioniert ; es resultierten

eine Fraktion von 18,2 g mit $Kp_{13}$ 80 °C (2-Ethylhexanol) und

eine Fraktion von 51,51 g mit $Kp_{0,4}$ 116-117 °C (Bis-(2-ethylhexyl)-carbonat).

Ausbeute : 90 % bezogen auf eingesetzten Harnstoff.

### Beispiel 2

12,0 g Harnstoff, 65,0 g 2-Ethylhexanol und 1,28 g Tetraphenylzinn wurden analog Beispiel 1 umgesetzt. Nach 4 Stunden Reaktionszeit betrug die Ausbeute an Kohlensäureester 82,0 %.

### Beispiel 3

9,0 g Harnstoff, 52,0 g 2-Ethylhexanol und 1,89 g Dibutylzinndilaurat wurden analog Beispiel 1 umgesetzt. Nach 8,5 Stunden Reaktionszeit wurden 52,6 % Kohlensäureester erhalten.

### Beispiel 4

9,0 g Harnstoff, 52,0 g 2-Ethylhexanol und 0,89 g Dibutyldimethoxyzinn wurden analog Beispiel 1 umgesetzt. Nach 9 Stunden Reaktionszeit betrug die Ausbeute an Kohlensäureester 88,6 %.

### Vergleichsbeispiel 1

9,0 g Harnstoff, 52,0 g 2-Ethylhexanol und 0,79 g Triphenylphosphin wurden analog Beispiel 1 umgesetzt. Nach 4 Stunden Reaktionszeit betrug die Ausbeute an Kohlensäureester 8,1 %.

### Vergleichsbeispiel 2

9,0 g Harnstoff, 52,0 g 2-Ethylhexanol und 0,36 g Dimethylphenylamin (N,N-Dimethylanilin) wurden analog Beispiel 1 umgesetzt. Nach 4 Stunden Reaktionszeit betrug die Ausbeute an Kohlensäureester 5,8 %.

### Vergleichsbeispiel 3

9,0 g Harnstoff, 52,0 g 2-Ethylhexanol und 0,85 g Triphenylphosphinoxid wurden analog Beispiel 1 umgesetzt. Nach 4 Stunden Reaktionszeit betrug die Ausbeute an Kohlensäureester 11,0 %.

## Ansprüche

1. Verfahren zur Herstellung von Kohlensäureestern aus Alkoholen und Harnstoffen im Molverhältnis von mindestens 2 : 1 (Alkoholgruppe : Harnstoff) oder aus Alkoholen und Urethanen im Molverhältnis von mindestens 1 : 1 (Alkoholgruppe : Urethangruppe) in Gegenwart von Katalysatoren, dadurch gekennzeichnet, daß man die Umsetzung bei Temperaturen zwischen 120 °C und 270 °C in Gegenwart eines Katalysators der Formel I

$$R^1_{n_1}-\overset{X_{n_2}}{\underset{(OH)_{n_4}}{M}}-(O-R^2)_{n_3} \qquad (I)$$

worin

M = Si, Ge oder Sn,
$R_1 = C_1$-$C_{18}$-Alkyl oder $C_6$-$C_{20}$-Aryl,
$R_2 = C_1$-$C_{18}$-Alkyl, $C_6$-$C_{20}$-Aryl oder $C_2$-$C_{18}$-Acyl,
X = Halogen, —SH, =O/2 oder =S/2,
$n_1$, $n_2$, $n_3$ und $n_4$ jeweils eine ganze Zahl von 0 bis 4, und
$n_1 + n_2 + n_3 + n_4 = 4$ ist,
wobei für M = Sn $n_1$ nicht O ist, und worin in Formel I außerdem die Summe.
$n_1 + n_2 + n_3 + n_4 = 2$ sein kann, wobei allerdings $n_2$ nicht 2 sein darf, und worin
in Formel I auch M = As sein kann, wobei die Summe aus $n_1 + n_2 + n_3 + n_4$ die Werte 3 oder 5 hat ; und worin
in Formel I auch M = S, Se oder Te sein kann, wobei die Summe aus $n_1 + n_2 + n_3 + n_4$ die Werte 4 oder 6 hat, durchführt.

2. Verfahren gemäß Ansprüche 1, dadurch gekennzeichnet, daß in Formel I X Cl, $R_1$ $C_1$-$C_8$-Alkyl oder Phenyl und $R_2$ $C_1$-$C_8$-Alkyl, Phenyl oder $-\underset{\underset{O}{\|}}{C}-(C_1$-$C_8)$-Alkyl sind.

3. Verfahren gemäß Ansprüche 1 und 2, zur Herstellung von Polycarbonaten, dadurch gekennzeichnet, daß man Dialkohole mit Harnstoffen im Molverhältnis 1 : 1 oder Dialkohole mit Urethanen im Molverhältnis Dialkohol : Urethangruppe 0,5 : 1 umsetzt.

4. Verfahren gemäß Ansprüche 1 bis 3, dadurch gekennzeichnet, daß man die Katalysatoren der Formel I in Mengen zwischen $10^{-3}$ Mol-% und 5 Mol-%, bezogen auf Mole Harnstoff oder Urethane, einsetzt.

## Claims

1. Process for the production of carbonic acid esters from alcohols and ureas in a molar ration of at least 2 : 1 (alcohol group : urea) or from alcohols and urethanes in a molar ratio of at least 1 : 1 (alcohol group : urethane group) in the presence of catalysts, characterized in that the reaction is carried out at temperatures in the range from 120 to 270 °C in the presence of a catalyst corresponding to the following formula

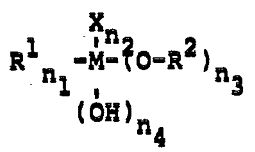

(I)

in which
M = Si, Ge or Sn,
$R_1 = C_1$-$C_{18}$-alkyl or $C_6$-$C_{20}$-aryl,
$R_2 = C_1$-$C_{18}$-alkyln $C_6$-$C_{20}$-aryl or $C_2$-$C_{18}$-acyl,
X = halogen, —SH, =O/2 or =S/2,
$n_1$, $n_2$, $n_3$ and $n_4$ are each an integer from 0 to 4 and
$n_1 + n_2 + n_3 + n_4 = 4$,
where M = Sn, $n_1 = 0$,
in addition to which the sum of $n_1 + n_2 + n_3 + n_4$ in formula I may amount to 2, although in that case $n_2$ cannot be 2 ;
in formula I, M may also represent As, in which case the sum of $n_1 + n_2 + n_3 + n_4$ has the value 3 or 5 ;
in formula I, M may also represent S, Se or Te, in which case the sum of $n_1 + n_2 + n_3 + n_4$ has the value 4 or 6.

2. A process as claimed in Claim 1, characterized in that, in formula I, X represents Cl, $R_1$ represents $C_1$-$C_8$-alkyl or phenyl and $R_2$ represents $C_1$-$C_8$-alkyl, phenyl or $-\underset{\underset{O}{\|}}{C}-(C_1$-$C_8)$-alkyl.

3. A process as claimed in Claims 1 and 2 for the production of polycarbonates, characterized in that dialcohols are reacted with ureas in a molar ratio of 1 : 1 or dialcohols are reacted with urethanes in a molar ratio of dialcohol to urethane group of 0,5 : 1.

4. A process as claimed in Claims 1 to 3, characterized in that the catalysts corresponding to formula I are used in quantities of from $10^{-3}$ mole percent and 5 mole percent, based on moles of urea or urethanes.

## Revendications

1. Procédé de production d'esters d'acide carbonique à partir d'alcools et d'urées dans un rapport molaire d'au moins 2 : 1 (groupe alcool : urée) ou à partir d'alcools et d'uréthannes dans un rapport molaire d'au moins 1 : 1 (groupe alcool : groupe uréthanne) en présence de catalyseurs, caractérisé en ce

qu'on conduit la réaction à des températures comprises entre 120 °C et 270 °C en présence d'un catalyseur de formule I

$$R^1_{n_1}-\underset{(OH)_{n_4}}{\overset{X_{n_2}}{M}}-(O-R^2)_{n_3} \qquad (I)$$

dans laquelle

M = Si, Ge ou Sn,

$R_1$ = alkyle en $C_1$ à $C_{18}$ ou aryle en $C_6$ à $C_{20}$,

$R_2$ = alkyle en $C_1$ à $C_{18}$, aryle en $C_6$ à $C_{20}$ ou acyle en $C_2$ à $C_{18}$,

X = halogène, —SH, = O/2 ou = S/2,

$n_1$, $n_2$, $n_3$ et $n_4$ représentent chacun un nombre entier de 0 à 4, et

$n_1 + n_2 + n_3 + n_4 = 4$,

au cas où M = Sn, $n_1$ n'est pas égal à 0 et en outre, dans la formule I, la somme $n_1 + n_2 + n_3 + n_4$ peut être égale à 2, auquel cas $n_2$ ne peut assurément pas être égal à 2, et dans la formule I, M peut aussi être égal à As, auquel cas la somme $n_1 + n_2 + n_3 + n_4$ a la valeur 3 ou 5 ; et dans la formule I, M peut aussi être égal à S, Se ou Te, auquel cas la somme $n_1 + n_2 + n_3 + n_4$ a la valeur 4 ou 6.

2. Procédé suivant la revendication 1, caractérisé en ce que dans la formule I, X représente Cl, $R_1$ représente un groupe alkyle en $C_1$ à $C_8$ ou phényle et $R_2$ est un groupe alkyle en $C_1$ à $C_8$, phényle ou $-\underset{\underset{O}{\|}}{C}-$(alkyle en $C_1$ à $C_8$).

3. Procédé suivant les revendications 1 et 2 pour la production de polycarbonates, caractérisé en ce qu'on fait réagir des dialcools avec des urées dans un rapport molaire de 1 : 1 ou des dialcools avec des uréthannes dans un rapport molaire dialcool : groupe uréthanne de 0,5 : 1.

4. Procédé suivant les revendications 1 à 3, caractérisé en ce qu'on utilise les catalyseurs de formule I en quantités comprises entre $10^{-3}$ et 5 moles %, par rapport aux moles d'urée ou d'uréthannes.